# EUROPEAN PATENT SPECIFICATION

(11) **EP 3 245 938 B1**
(45) Date of publication and mention of the grant of the patent: **01.05.2019**
(21) Application number: 17171452.0
(22) Date of filing: 17.05.2017
(51) Int. Cl.: A61B 1/12, A61B 1/00

(54) **APPARATUS AND METHOD TO IDENTIFY ENDOSCOPE TYPE AND PROVIDE TAILORED REPROCESSING**
VORRICHTUNG UND VERFAHREN ZUM IDENTIFIZIEREN VON ENDOSKOPEN UND MASSGESCHNEIDERTE BEREITSTELLUNG VON WIEDERVERARBEITUNG
APPAREIL ET PROCÉDÉ D'IDENTIFICATION DE TYPE D'ENDOSCOPE ET DE FOURNITURE DE RETRAITEMENT PERSONNALISÉ

(30) Priority: 18.05.2016 US 201615157650
(43) Date of publication of application: 22.11.2017
(73) Proprietor: Ethicon, Inc., Somerville, NJ 08876 (US)
(72) Inventor: YANG, Sungwook, Irvine, CA California 92618 (US); WILLIAMS, Harold R., Laguna Beach, CA California 92651 (US)
(74) Representative: Carpmaels & Ransford LLP

(56) References cited:
- EP-A1- 2 060 276
- US-A1- 2005 065 405
- US-A1- 2009 220 377
- US-A1- 2014 166 059

## Description

### BACKGROUND

The below discussion relates to the reprocessing (i.e., decontamination) of endoscopes and other instruments that are used in medical procedures. In particular, the below discussion relates to an apparatus and a method that may be used to reprocess a medical device such as an endoscope after the medical device has been used in a first medical procedure, such that the medical device may be safely used in a subsequent medical procedure. While the below discussion will speak mainly in terms of an endoscope, it should be understood that the discussion may also equally apply to certain other medical devices.

An endoscope may have one or more working channels or lumens extending along at least a portion of the length of the endoscope. Such channels may be configured to provide a pathway for passage of other medical devices, etc., into an anatomical region within a patient. These channels may be difficult to clean and/or disinfect using certain primitive cleaning and/or disinfecting techniques. Thus, the endoscope may be placed in a reprocessing system that is particularly configured to clean endoscopes, including the channels within endoscopes. Such an endoscope reprocessing system may wash and disinfect the endoscope. Such an endoscope reprocessing system may include a basin that is configured to receive the endoscope, with a pump that flows cleaning fluids over the exterior of the endoscope within the basin. The system may also include ports that couple with the working channels of the endoscope and associated pumps that flow cleaning fluids through the working channels of the endoscope. The process executed by such a dedicated endoscope reprocessing system may include a detergent washing cycle, followed by a rinsing cycle, followed by a sterilization or disinfection cycle, followed by another rinsing cycle. The sterilization or disinfection cycle may employ disinfection solution and water rinses. The process may optionally include an alcohol flush to aid displacement of water. A rinsing cycle may be followed by an air flush for drying and storage.

Examples of systems and methods that may be used to reprocess a used endoscope are described in U.S. Pat. No. 6,986,736, entitled "Automated Endoscope Reprocessor Connection with Integrity Testing," issued January 17, 2006; U.S. Pat. No. 7,479,257, entitled "Automated Endoscope Reprocessor Solution Testing," issued January 20, 2009; U.S. Pat. No. 7,686,761, entitled "Method of Detecting Proper Connection of an Endoscope to an Endoscope Reprocessor," issued March 30, 2010; and U.S. Pat. No. 8,246,909, entitled "Automated Endoscope Reprocessor Germicide Concentration Monitoring System and Method," issued August 21, 2012. An example of a commercially available endoscope reprocessing system is the EVOTECH® Endoscope Cleaner and Reprocessor (ECR) by Advanced Sterilization Products of Irvine, California. US 2009/0220377 A1 discloses an endoscope washing and disinfecting apparatus that includes: a fluid supply unit that supplies fluid for washing and disinfecting, an electromagnetic valve provided in each of a plurality of connecting channels which are connected to a plurality of channels of an endoscope, a single flow rate meter provided between the fluid supply unit and the electromagnetic valve, and a flow rate limiting section for limiting flow rate so that the flow rate falls within a flow rate measurement range in which measurement is possible.

Document US5738824 A discloses an apparatus and method for identification of a medical instrument based on the fluid volume flowing through all the channels and capillary tubes of an instrument in a predetermined period of time and selection of an appropriate cleaning program for the identified instrument.

While a variety of systems and methods have been made and used to reprocess medical devices, it is believed that no one prior to the inventor(s) has made or used the technology as described herein.

The present invention provides apparatus and methods as recited in the claims.

### BRIEF DESCRIPTION OF THE DRAWINGS

It is believed the present invention will be better understood from the following description of certain examples taken in conjunction with the accompanying drawings, in which like reference numerals identify the same elements and in which:
FIG. 1 depicts a front elevational view of an exemplary reprocessing system;
FIG. 2 depicts a schematic diagram of the reprocessing system of FIG. 1, with only a single decontamination basin shown for clarity;
FIG. 3 depicts a cross-sectional side view of proximal and distal portions of an endoscope that may be decontaminated using the reprocessing system of FIG. 1;
FIG. 4 depicts a diagrammatical view of an exemplary endoscope and detection system that may be incorporated into the reprocessing system of FIG. 1; and
FIG. 5 depicts a flow chart showing an exemplary method that may be performed by an operator using the detection system of FIG. 4 to identify an endoscope; and
FIG. 6 depicts a flow chart showing an exemplary method that may be performed by the detection system of FIG. 4 to identify an endoscope.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Medical Device Reprocessing Apparatus

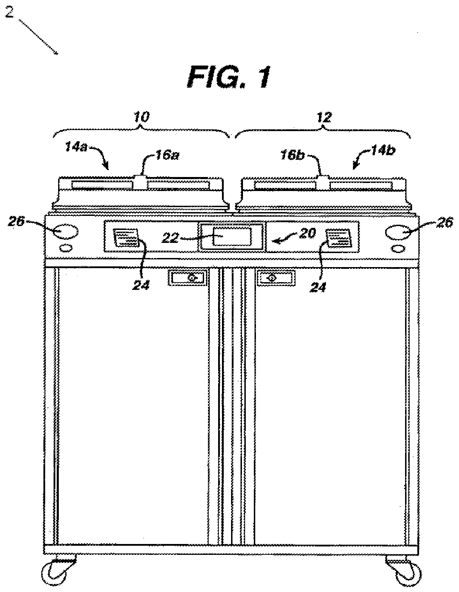

FIGS. 1-2 show an exemplary reprocessing system (2) that may be used to decontaminate endoscopes and other medical devices that include channels or lumens formed therethrough. System (2) of this example generally includes a first station (10) and a second station (12). Stations (10, 12) are at least substantially similar in all respects to provide for the decontamination of two different medical devices simultaneously or in series. First and second decontamination basins (14a, 14b) receive the contaminated devices. Each basin (14a, 14b) is selectively sealed by a respective lid (16a, 16b). In the present example, lids (16a, 16b) cooperate with respective basins (14a, 14b) to provide a microbe-blocking relationship to prevent the entrance of environmental microbes into basins (14a, 14b) during decontamination operations. By way of example only, lids (16a, 16b) may include a microbe removal or HEPA air filter formed therein for venting.

A control system (20) includes one or more microcontrollers, such as a programmable logic controller (PLC), for controlling decontamination and user interface operations. Although one control system (20) is shown herein as controlling both decontamination stations (10, 12), those skilled in the art will recognize that each station (10, 12) can include a dedicated control system. A visual display (22) displays decontamination parameters and machine conditions for an operator, and at least one printer (24) prints a hard copy output of the decontamination parameters for a record to be filed or attached to the decontaminated device or its storage packaging. It should be understood that printer (24) is merely optional. In some versions, visual display (22) is combined with a touch screen input device. In addition or in the alternative, a keypad and/or other user input feature is provided for input of decontamination process parameters and for machine control. Other visual gauges (26) such as pressure meters and the like provide digital or analog output of decontamination or medical device leak testing data.

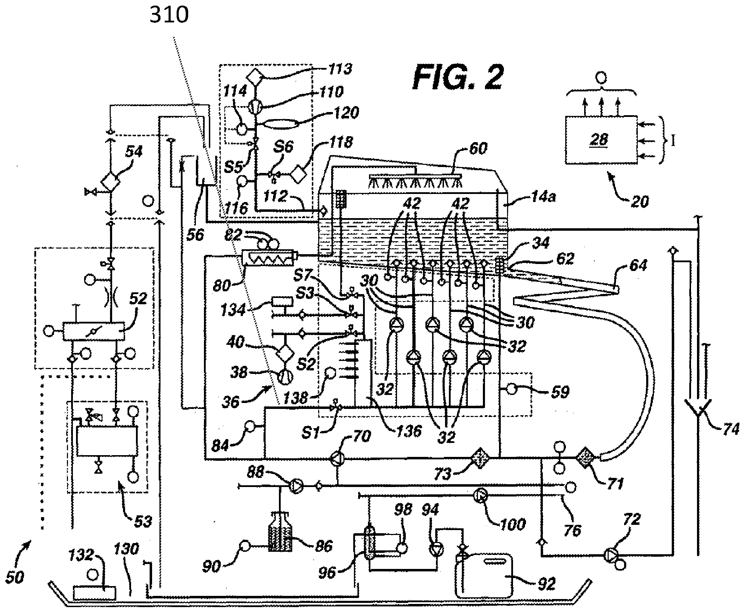

FIG. 2 diagrammatically illustrates just one decontamination station (10) of reprocessing system (2), but those skilled in the art will recognize that decontamination station (12) may be configured and operable just like decontamination station (10). It should also be understood that reprocessing system (2) may be provided with just one single decontamination station (10, 12) or more than two decontamination stations (10, 12).

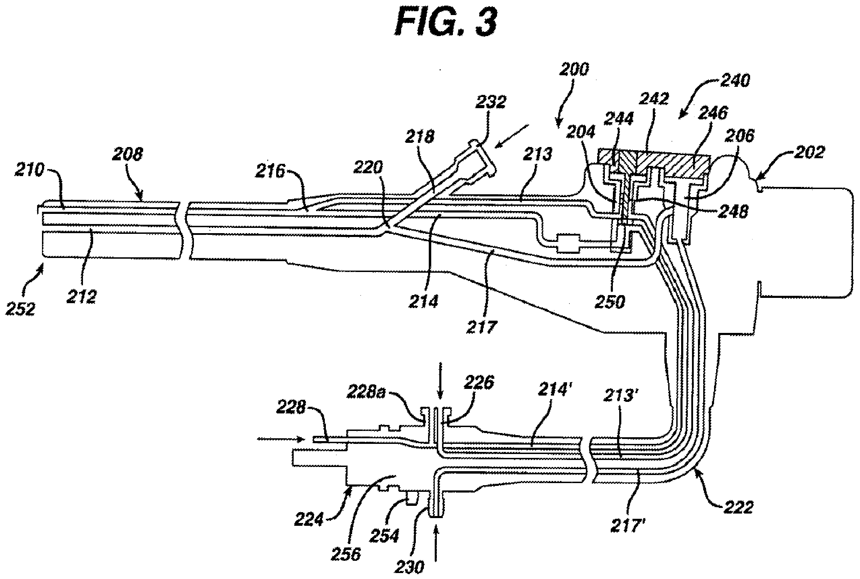

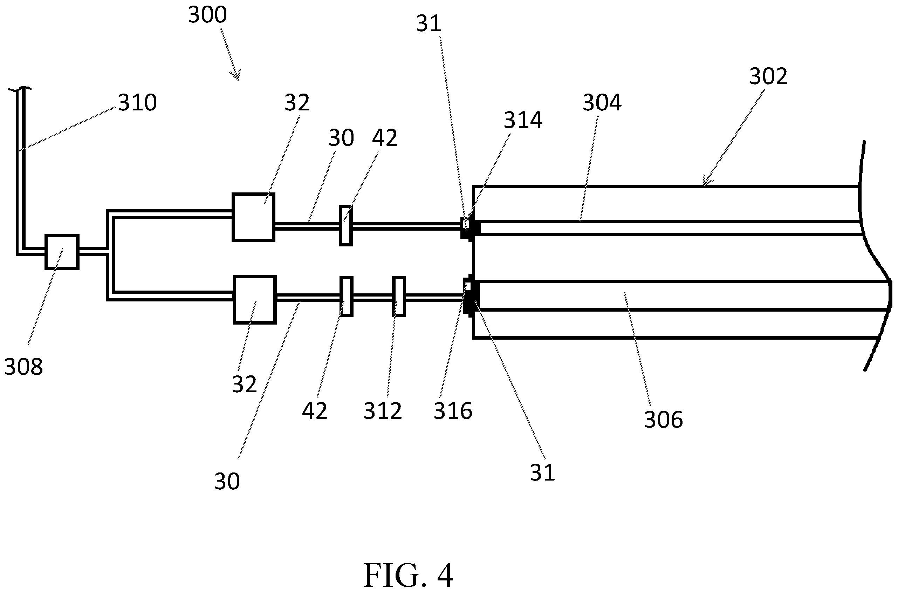

As noted above, decontamination basin (14a) receives an endoscope (200) (see FIG. 3) or other medical device therein for decontamination. Any internal channels of endoscope (200) are connected with flush lines (30). In some versions of reprocessing system (2), and as will be described in greater detail below with reference to FIG. 4, internal channels of endoscope (200) are connected with flush lines (30) via a plurality of connectors (31) (see FIG. 4) at the terminal end of each flush line (30). Each connector (31) may be connected to a corresponding and complementary connector (not shown) of the endoscope (200); or may be inserted directly into the opening of the associated channel or lumen of the endoscope (200) in a press fit or a screw style engagement. In some other versions of reprocessing system (2) flush lines (30) are coupled with endoscope (200) via a single connector (not shown) with multiple lumens or channels defined therein and coupled with a corresponding flush lines (30). Each flush line (30) is connected to an outlet of a corresponding pump (32), such that each flush line (30) has a dedicated pump (32) in this example. Pumps (32) of the present example comprise peristaltic pumps that pump fluid, such as liquid and air, through the flush lines (30) and any internal channels of endoscope (200). Alternatively, any other suitable kind of pump(s) may be used. In the present example, pumps (32) can either draw liquid from basin (14a) through a filtered drain (34) and a valve (S1); or draw decontaminated air from an air supply system (36) through a valve (S2). Air supply system (36) of the present example includes a pump (38) and a microbe removal air filter (40) that filters microbes from an incoming air stream.

A pressure switch or sensor (42) is in fluid communication with each flush line (30) for sensing excessive pressure in the flush line. Any excessive pressure or lack of flow sensed may be indicative of a partial or complete blockage (e.g., by bodily tissue or dried bodily fluids) in an endoscope (200) channel to which the relevant flush line (30) is connected. The isolation of each flush line (30) relative to the other flush lines (30) allows the particular blocked channel to be easily identified and isolated, depending upon which sensor (42) senses excessive pressure or lack of flow.

Basin (14a) is in fluid communication with a water source (50), such as a utility or tap water connection including hot and cold inlets, and a mixing valve (52) flowing into a break tank (56). A microbe removal filter (54), such as a 0.2 µm or smaller absolute pore size filter, decontaminates the incoming water, which is delivered into break tank (56) through the air gap to prevent backflow. A sensor (59) monitors liquid levels within basin (14a). An optional water heater (53) can be provided if an appropriate source of hot water is not available. The condition of filter (54) can be monitored by directly monitoring the flow rate of water therethrough or indirectly by monitoring the basin fill time using a float switch or the like. When the flow rate drops below a select threshold, this indicates a partially clogged filter element that requires replacement.

A basin drain (62) drains liquid from basin (14a) through an enlarged helical tube (64) into which elongated portions of endoscope (200) can be inserted. Drain (62) is in fluid communication with a recirculation pump (70) and a drain pump (72). Recirculation pump (70) recirculates liquid from basin drain (62) to a spray nozzle assembly (60), which sprays the liquid into basin (14a) and onto endoscope (200). A coarse screen (71) and a fine screen (73) filter out particles in the recirculating fluid. Drain pump (72) pumps liquid from basin drain (62) to a utility drain (74). A level sensor (76) monitors the flow of liquid from pump (72) to utility drain (74). Pumps (70, 72) can be simultaneously operated such that liquid is sprayed into basin (14a) while basin (14a) is being drained, to encourage the flow of residue out of basin (14a) and off of endoscope (200). Of course, a single pump and a valve assembly could replace dual pumps (70, 72).

An inline heater (80), with temperature sensors (82), upstream of recirculation pump (70), heats the liquid to optimum temperatures for cleaning and/or disinfection. A pressure switch or sensor (84) measures pressure downstream of circulation pump (70). In some variations, a flow sensor is used instead of pressure sensor (84), to measure fluid flow downstream of circulation pump (70). Detergent solution (86) is metered into the flow downstream of circulation pump (70) via a metering pump (88). A float switch (90) indicates the level of detergent (86) available. Disinfectant (92) is metered into the flow upstream of circulation pump (70) via a metering pump (94). To more accurately meter disinfectant (92), a dispensing pump (94) fills a metering pre-chamber (96) under control of a fluid level switch (98) and control system (20). By way of example only, disinfectant (92) may comprise CIDEX® Activated Glutaraldehyde Solution by Advanced Sterilization Products of Irvine, California. By way of further example only, disinfectant (92) may comprise ortho-phthalaldehyde (OPA) solution.

Some endoscopes (200) include a flexible outer housing or sheath surrounding the individual tubular members and the like that form the interior channels and other parts of endoscope (200). This housing defines a closed interior space, which is isolated from patient tissues and fluids during medical procedures. It may be important that the sheath be maintained intact, without cuts or other holes that would allow contamination of the interior space beneath the sheath. Therefore, reprocessing system (2) of the present example includes means for testing the integrity of such a sheath. In particular, an air pump (e.g., pump (38) or another pump (110)) pressurizes the interior space defined by the sheath of endoscope (200) through a conduit (112) and a valve (S5). In the present example, a HEPA or other microbe-removing filter (113) removes microbes from the pressurizing air. A pressure regulator (114) prevents accidental over pressurization of the sheath. Upon full pressurization, valve (S5) is closed and a pressure sensor (116) looks for a drop in pressure in conduit (112), which would indicate the escape of air through the sheath of endoscope (200). A valve (S6) selectively vents conduit (112) and the sheath of endoscope (200) through an optional filter (118) when the testing procedure is complete. An air buffer (120) smoothes out pulsation of pressure from air pump (110).

In the present example, each station (10, 12) also contains a drip basin (130) and spill sensor (132) to alert the operator to potential leaks.

An alcohol supply (134), controlled by a valve (S3), can supply alcohol to channel pumps (32) after rinsing steps, to assist in removing water from channels (210, 212, 213, 214, 217, 218) of endoscope (200).

Flow rates in supply lines (30) can be monitored via channel pumps (32) and pressure sensors (42). If one of pressure sensors (42) detects too high a pressure, the associated pump (32) is deactivated. The flow rate of pump (32) and its activated duration time provide a reasonable indication of the flow rate in an associated line (30). These flow rates are monitored during the process to check for blockages in any of the channels of endoscope (200). Alternatively, the decay in the pressure from the time pump (32) cycles off can also be used to estimate the flow rate, with faster decay rates being associated with higher flow rates.

A more accurate measurement of flow rate in an individual channel may be desirable to detect more subtle blockages. To that end, a metering tube (136) having a plurality of level indicating sensors (138) fluidly connects to the inputs of channel pumps (32). In some versions, a reference connection is provided at a low point in metering tube (136) and a plurality of sensors (138) are arranged vertically above the reference connection. By passing a current from the reference point through the fluid to sensors (138), it can be determined which sensors (138) are immersed and therefore determine the level within metering tube (136). In addition or in the alternative, any other suitable components and techniques may be used to sense fluid levels. By shutting valve (S1) and opening a vent valve (S7), channel pumps (32) draw exclusively from metering tube (136). The amount of fluid being drawn can be very accurately determined based upon sensors (138). By running each channel pump (32) in isolation, the flow therethrough can be accurately determined based upon the time and the volume of fluid emptied from metering tube (136).

In addition to the input and output devices described above, all of the electrical and electromechanical devices shown are operatively connected to and controlled by control system (20). Specifically, and without limitation, switches and sensors (42, 59, 76, 84, 90, 98, 114, 116, 132 136) provide input (I) to microcontroller (28), which controls the cleaning and/or disinfection cycles and other machine operations in accordance therewith. For example, microcontroller (28) includes outputs (O) that are operatively connected to pumps (32, 38, 70, 72, 88, 94, 100, 110), valves (S1, S2, S3, S5, S6, S7), and heater (80) to control these devices for effective cleaning and/or disinfection cycles and other operations.

As shown in FIG. 3, endoscope (200) has a head part (202). Head part (202) includes openings (204, 206) formed therein. During normal use of endoscope (200), an air/water valve (not shown) and a suction valve (not shown) are arranged in openings (204, 206). A flexible insertion tube (208) is attached to head part (202). A combined air/water channel (210) and a combined suction/biopsy channel (212) are accommodated in insertion tube (208). A separate air channel (213) and water channel (214) are also arranged in head part (202) and merge into air/water channel (210) at the location of a joining point (216). It will be appreciated that the term "joining point" as used herein refers to an intersecting junction rather than being limited to a geometrical point and, the terms may be used interchangeably. Furthermore, a separate suction channel (217) and biopsy channel (218) are accommodated in head part (202) and merge into suction/biopsy channel (212) at the location of a joining point (220).

In head part (202), air channel (213) and water channel (214) open into opening (204) for the air/water valve (not shown). Suction channel (217) opens into opening (206) for the suction valve (not shown). Furthermore, a flexible feed hose (222) connects to head part (202) and accommodates channels (213', 214', 217'), which are connected to air channel (213), water channel (214), and suction channel (217) via respective openings (204, 206). In practice, feed hose (222) may also be referred to as the light-conductor casing. The mutually connecting air channels (213, 213') will collectively be referred to below as air channel (213). The mutually connecting water channels (214, 214') will collectively be referred to below as water channel (214). The mutually connecting suction channels (217, 217') will collectively be referred to below as suction channel (217). A connection (226) for air channel (213), connections (228, 228a) for water channel (214), and a connection (230) for suction channel (217) are arranged on the end section (224) (also referred to as the light conductor connector) of flexible hose (222). When the connection (226) is in use, connection (228a) is closed off. A connection (232) for biopsy channel (218) is arranged on head part (202).

A channel separator (240) is shown inserted into openings (204, 206). Channel separator (240) comprises a body (242) and plug members (244, 246), which occlude respective openings (204, 206). A coaxial insert (248) on plug member (244) extends inwardly of opening (204) and terminates in an annular flange (250), which occludes a portion of opening (204) to separate channel (213) from channel (214). By connecting lines (30) to openings (226, 228, 228a, 230, 232), liquid for cleaning and disinfection can be flowed through endoscope channels (213, 214, 217, 218) and out of a distal tip (252) of endoscope (200) via channels (210, 212). Channel separator (240) ensures that such liquid flows all the way through endoscope (200) without leaking out of openings (204, 206); and isolates channels (213, 214) from each other so that each channel (213, 214) has its own independent flow path. One of skill in the art will appreciate that various endoscopes having differing arrangements of channels and openings may require modifications to channel separator (240) to accommodate such differences while occluding ports in head (202) and keeping channels separated from each other so that each channel can be flushed independently of the other channels. Otherwise, a blockage in one channel might merely redirect flow to a connected unblocked channel.

A leakage port (254) on end section (224) leads into an interior portion (256) of endoscope (200) and is used to check for the physical integrity thereof, namely to ensure that no leakage has formed between any of the channels and the interior (256) or from the exterior to the interior (256).

### II. Exemplary Medical Device Reprocessing Method

In an exemplary use of reprocessing system (2), an operator may start by actuating a foot pedal (not shown) to open basin lid (16a). Each lid (16a, 16b) may have its own foot pedal. In some versions, once pressure is removed from the foot pedal, the motion of lid (16a, 16b) stops. With lid (16a) open, the operator inserts insertion tube (208) of endoscope (200) into helical circulation tube (64). End section (224) and head section (202) of endoscope (200) are situated within basin (14a), with feed hose (222) coiled within basin (14a) with as wide a diameter as possible. Next, flush lines (30) are attached to respective endoscope openings (226, 228, 228a, 230, 232). Air line (112) is also connected to connector (254). In some versions, flush lines (30) are color coded, and guide located on station (10) provides a reference for the color-coded connections.

Depending on the customer-selectable configuration, control system (20) may prompt the operator to enter a user code, patient ID, endoscope code, and/or specialist code. This information may be entered manually (e.g., through touch screen (22)), automatically (e.g., by using an attached barcode wand), or in any other suitable fashion. Further, as will be discussed in greater detail below, endoscope information such as the type and style of endoscope may be automatically detected by reprocessing system (2). With the information entered (if required), the operator may then close lid (16a). In some versions, closing lid (16a) requires the operator to press a hardware button and a touch-screen (22) button simultaneously to provide a fail-safe mechanism for preventing the operator's hands from being caught or pinched by the closing basin lid (16a). If either the hardware button or software button is released while lid (16a) is in the process of closing, the motion of lid (16a) stops.

Once lid (16a) is closed, the operator presses a button on touch-screen (22) to begin the washing/disinfection process. At the start of the washing/disinfection process, air pump (38) is activated and pressure within the body of endoscope (200) is monitored. When pressure reaches a predetermined level (e.g., 250 mbar), pump (38) is deactivated, and the pressure is allowed to stabilize for a certain stabilization period (e.g., 6 seconds). If pressure has not reached a certain pressure (e.g., 250 mbar) in a certain time period (e.g., 45 seconds), the program is stopped and the operator is notified of a leak. If pressure drops below a threshold (e.g., less than 100 mbar) during the stabilization period, the program is stopped and the operator is notified of the condition. Once the pressure has stabilized, the pressure drop is monitored over the course of a certain duration (e.g., 60 seconds). If pressure drop is faster than a predetermined rate (e.g., more than 10 mbar within 60 seconds), the program is stopped and the operator is notified of the condition. If the pressure drop is slower than a predetermined rate (e.g., less than 10 mbar in 60 seconds), reprocessing system (2) continues with the next step. A slight positive pressure is held within the body of endoscope (200) during the rest of the process to prevent fluids from leaking in.

A second leak test checks the adequacy of connection to the various ports (226, 228, 228a, 230, 232) and the proper placement of channel separator (240). A quantity of water is admitted to basin (14a) so as to submerge the distal end of endoscope (200) in helical tube (64). Valve (S1) is closed and valve (S7) opened; and pumps (32) are run in reverse to draw a vacuum and to ultimately draw liquid into endoscope channels (210, 212). Pressure sensors (42) are monitored to make sure that the pressure in any one channel (210, 212) does not drop and/or raise by more than a predetermined amount in a given time frame. If it does, it likely indicates that one of the connections was not made correctly and air is leaking into channel (210, 212). In any event, in the presence of an unacceptable pressure drop, control system (20) will cancel the cycle and indicate a likely faulty connection, preferably with an indication of which channel (210, 212) failed.

In the event that the leak tests are passed, reprocessing system (2) continues with a pre-rinse cycle. The purpose of this step is to flush water through channels (210, 212, 213, 214,217,218) to remove waste material prior to washing and disinfecting endoscope (200). To initiate the pre-rinse cycle, basin (14a) is filled with filtered water and the water level is detected by pressure sensor (59) below basin (14a). The water is pumped via pumps (32) through the interior of channels (210, 212, 213, 214, 217, 218), directly to drain (74). This water is not recirculated around the exterior surfaces of endoscope 200 during this stage. As the water is being pumped through channels (210, 212, 213, 214, 217, 218), drain pump (72) is activated to ensure that basin (14a) is also emptied. Drain pump (72) will be turned off when drain switch (76) detects that the drain process is complete. During the draining process, sterile air is blown via air pump (38) through all endoscope channels (210, 212, 213, 214, 217, 218) simultaneously, to minimize potential carryover.

Once the pre-rinse cycle is complete, reprocessing system (2) continues with a wash cycle. To begin the wash cycle, basin (14a) is filled with warm water (e.g., approximately 35°C). Water temperature is controlled by controlling the mix of heated and unheated water. The water level is detected by pressure sensor (59). Reprocessing system (2) then adds enzymatic detergent to the water circulating in reprocessing system (2) by means of peristaltic metering pump (88). The volume is controlled by controlling the delivery time, pump speed, and inner diameter of the tubing of pump (88). Detergent solution (86) is actively pumped throughout the internal endoscope channels (210,212,213,214,217,218) and over the outer surface of endoscope (200) for a predetermined time period (e.g., from one to five minutes, or more particularly about three minutes), by channel pumps (32) and external circulation pump (70). Inline heater (80) keeps the temperature at a predetermined temperature (e.g., approximately about 35° C).

After detergent solution (86) has been circulating for a certain period of time (e.g., a couple of minutes), the flow rate through channels (210, 212, 213, 214, 217, 218) is measured. If the flow rate through any channel (210, 212, 213, 214, 217, 218) is less than a predetermined rate for that channel (210, 212, 213, 214, 217, 218), the channel (210, 212, 213, 214, 217, 218) is identified as blocked, the program is stopped, and the operator is notified of the condition. Peristaltic pumps (32) are run at their predetermined flow rates and cycle off in the presence of unacceptably high pressure readings at the associated pressure sensor (42). If a channel (210, 212, 213, 214, 217, 218) is blocked, the predetermined flow rate will trigger pressure sensor (42), indicating the inability to adequately pass this flow rate. As pumps (32) are peristaltic in the present example, their operating flow rate combined with the percentage of time they are cycled off due to pressure will provide the actual flow rate. The flow rate can also be estimated based upon the decay of the pressure from the time pump (32) cycles off.

At the end of the wash cycle, drain pump (72) is activated to remove detergent solution (86) from basin (14a) and channels (210, 212, 213, 214, 217, 218). Drain pump (72) turns off when drain level sensor (76) indicates that drainage is complete. During the drain process, sterile air is blown through all channels (210, 212, 213, 214, 217, 218) of endoscope (200) simultaneously to minimize potential carryover.

After the wash cycle is complete, reprocessing system (2) begins a rinse cycle. To initiate this rinse cycle, basin (14a) is again filled with warm water (e.g., at approximately 35° C.). Water temperature is controlled by controlling the mix of heated and unheated water. The water level is detected by pressure sensor (59). The rinse water is circulated within channels (210, 212, 213, 214, 217, 218) of endoscope (200) via channel pumps (32); and over the exterior of endoscope (200) via circulation pump (70) and sprinkler arm (60) for a certain period of time (e.g., one minute). As rinse water is pumped through channels (210, 212, 213, 214, 217, 218), the flow rate through channels (210, 212, 213, 214, 217, 218) is measured and if it falls below the predetermined rate for any given channel (210, 212, 213, 214, 217, 218), that channel (210, 212, 213, 214, 217, 218) is identified as blocked, the program is stopped, and the operator is notified of the condition.

At the end of the rinse cycle, drain pump (72) is activated to remove the rinse water from basin (14a) and channels (210, 212, 213, 214, 217, 218). Drain pump (72) turns off when drain level sensor (76) indicates that drainage is complete. During the drain process, sterile air is blown through all channels (210, 212, 213, 214, 217, 218) of endoscope (200) simultaneously to minimize potential carryover. In some versions, the above-described rinsing and draining cycles are repeated at least once again, to ensure maximum rinsing of detergent solution (86) from the surfaces of endoscope (200) and basin (14a).

After reprocessing system (2) has completed the desired number of rinsing and drying cycles, reprocessing system (2) proceeds to a disinfection cycle. To initiate the disinfection cycle, basin (14a) is filled with very warm water (e.g., at approximately 53° C.). Water temperature is controlled by controlling the mix of heated and unheated water. The water level is detected by pressure sensor (59). During the filling process, channel pumps (32) are off in order to ensure that the disinfectant solution (92) in basin (14a) is at the in-use concentration prior to circulating through channels (210, 212, 213, 214, 217, 218) of endoscope (200).

Next, a measured volume of disinfection solution (92) is drawn from disinfectant metering pre-chamber (96) and delivered into the water in basin (14a) via metering pump (100). The volume of disinfection solution (92) is controlled by the positioning of fill level switch (98) relative to the bottom of metering pre-chamber (96). Metering pre-chamber (96) is filled until fill level switch (98) detects liquid. Disinfection solution (92) is drawn from metering pre-chamber (96) until the level of disinfection solution (92) in metering pre-chamber (96) is just below the tip of metering pre-chamber (96). After the necessary volume is dispensed, metering pre-chamber (96) is refilled from the bottle of disinfection solution (92). Disinfection solution (92) is not added until basin (14a) is filled, so that in case of a water supply problem, concentrated disinfectant is not left on endoscope (200) with no water to rinse it. While disinfection solution (92) is being added, channel pumps (32) are off in order to ensure that disinfection solution (92) in basin (14a) is at the desired in-use concentration prior to circulating through channels (210, 212, 213, 214, 217, 218) of endoscope (200).

The in-use disinfectant solution (92) is actively pumped throughout internal channels (210, 212, 213, 214, 217, 218) by pumps (32) and over the outer surface of endoscope (200) by circulation pump (70). This may be done for any suitable duration (e.g., at least 5 minutes). The temperature of the disinfection solution (92) may be controlled by in-line heater (80) to stay at a consistent temperature (e.g., about 52.5° C). During the disinfection process, flow through each channel (210, 212, 213, 214, 217, 218) of endoscope (200) is verified by timing the delivering a measured quantity of solution through channel (210, 212, 213, 214, 217, 218). Valve (S1) is closed, and valve (S7) opened, and in turn each channel pump (32) delivers a predetermined volume to its associated channel (210, 212, 213, 214, 217, 218) from metering tube (136). This volume and the time it takes to deliver the volume, provides a very accurate flow rate through the channel (210, 212, 213, 214, 217, 218). Anomalies in the flow rate from what is expected for a channel (210, 212, 213, 214, 217, 218) of that diameter and length are flagged by control system (20) and the process stopped. As in-use disinfection solution (92) is pumped through channels (210, 212, 213, 214, 217, 218), the flow rate through channels (210, 212, 213, 214, 217, 218) is also measured as described above.

At the end of the disinfection cycle, drain pump (72) is activated to remove disinfectant (92) solution from basin (14a) and channels (210, 212, 213, 214, 217, 218). During the draining process, sterile air is blown through all channels (210, 212, 213, 214, 217, 218) of endoscope (200) simultaneously to minimize potential carryover.

After disinfection solution (92) has been drained from basin (14a), reprocessing system (2) begins a final rinse cycle. To initiate this cycle, basin (14a) is filled with sterile warm water (e.g., at approximately 45° C) that has been passed through a filter (e.g., a 0.2µm filter). The rinse water is circulated within channels (210, 212,213, 214,217,218) by pumps (32); and over the exterior of endoscope (200) via circulation pump (70) and sprinkler arm 60) for a suitable duration (e.g., 1 minute). As rinse water is pumped through channels (210, 212, 213, 214, 217, 218), the flow rate through channels (210, 212, 213, 214, 217, 218) is measured as described above. Drain pump (72) is activated to remove the rinse water from basin (14a) and channels (210, 212, 213, 214, 217, 218). During the draining process, sterile air is blown through all channels (210, 212, 213, 214, 217, 218) of endoscope (200) simultaneously to minimize potential carryover. In some versions, the above-described rinsing and draining cycles are repeated at least two more times, to ensure maximum rinsing of disinfection solution (92) residuals from the surfaces of endoscope (200) and basin (14a).

After the final rinse cycle is complete, reprocessing system (2) begins a final leak test. In particular, reprocessing system (2) pressurizes the body of endoscope (200) and measures the leak rate as described above. If the final leak test is successful, reprocessing system (2) indicates the successful completion of the cycles via touch-screen (22). From the time of program completion to the time at which lid (16a) is opened, pressure within the body of endoscope (200) is normalized to atmospheric pressure by opening vent valve (S5) at a predetermined rate (e.g., valve (S5) opened for 10 seconds every minute).

Depending on customer-selected configuration, reprocessing system (2) may prevent lid (16a) from being opened until a valid user identification code is entered. Information about the completed program, including the user ID, endoscope ID, specialist ID, and patient ID are stored along with the sensor data obtained throughout the program. If a printer is connected to reprocessing system (2), and if requested by the operator, a record of the disinfection program will be printed. Once a valid user identification code has been entered, lid (16a) may be opened (e.g., using the foot pedal as described above). Endoscope (200) is then disconnected from flush lines (30) and removed from basin (14a). Lid (16a) can then be closed using both the hardware and software buttons as described above.

### III. Exemplary Automatic Detection System

Those of ordinary skill in the art will recognize that a variety of kinds of endoscopes exist, and that different kinds of endoscopes will have different kinds of configurations that may present unique challenges for a system such as reprocessing system (2). One of the most meaningful differences between various endoscope (200) types may be in the different configurations of endoscope channels (213, 214, 217, 218). Different endoscope channel (213, 214, 217, 218) configurations may warrant different durations of fluid flow through each line (30) and/or other variations in cleaning/disinfecting routines. For instance, a specific type of endoscope, referred to as a duodenoscope, may be formed with an elevator channel. The elevator channel may contain and guide a control wire that is used to manipulate a distally mounted device (e.g., a camera or other instrument). Due to the elevator channel having a very small opening or cross-sectional area, and further due to the presence of a control wire in the elevator channel, an elevator channel require additional time for flowing and purging to assure that the disinfectant reaches the distal end of the elevator channel for the duration of the disinfection time.

Some versions of reprocessing system (2) may enable the operator to select an endoscope (200) type from a list of endoscope (200) types, and reprocessing system (2) may make adjustments to the cleaning and disinfecting cycles to best suit the cycles to the particular endoscope (200) type based on the endoscope type (200) as identified by the operator. However, this may present room for operator error, as the operator may mistakenly identify the wrong endoscope (200) type. If the operator selects the wrong endoscope (200) type, reprocessing system (2) might not clean or disinfect the endoscope (200) to an optimal degree. For instance, sub-optimal disinfection of a duodenoscope with an open elevator channel may occur when a user does not recognize the device is a duodenoscope and processes the device as a non-duodenoscope. Similarly, sub-optimal disinfection may occur when the user does not realize, or cannot determine, that the duodenoscope is an open elevator channel duodenoscope.

Moreover, versions of reprocessing system (2) may enable the operator to select an endoscope type from a list of endoscope types would require reprocessing system (2) to have a current, up-to-date listing of endoscope (200) types. This would require updating when new endoscope (200) types are introduced.

To avoid the risk of user error in identifying an endoscope (200) type, which may lead to sub-optimal disinfection of endoscopes (200), and to avoid the need to have an exhaustive list of current endoscope (200) types, reprocessing system (2) may include a detection system (300) for automatically detecting the type of endoscope (200) disposed in decontamination basin (14a, 14b). After the type of endoscope (200) is determined, detection system (300) is configured to retrieve or generate a processing profile associated with the endoscope (200) and thereafter clean and disinfect the endoscope (200) according to the corresponding processing profile. Different processing profiles may provide different cleaning cycles and/or disinfecting cycles based on the type of endoscope (200) that has been automatically detected. For instance, when reprocessing system (2) detects that endoscope (200) is a duodenoscope having an open elevator channel, reprocessing system (2) may select a processing profile that provides enhanced disinfection flow to ensure that the distal end of the open elevator channel is properly disinfected. Other kinds of unique processing profile aspects that may be appropriate based on the detected type of endoscope (200) will be apparent to those of ordinary skill in the art in view of the teachings herein.

FIG. 4 shows detection system (300) that may be incorporated into reprocessing system (2) and attached to a corresponding generalized endoscope (302) having an exemplary first channel (304) and an exemplary second channel (306). While only two channels (304, 306) are provided in this particular example, it should be understood that the teachings may be readily applied to other endoscopes (200) that have more than two channels (213, 214, 217, 218). In addition, while detection system (300) is described herein in the context of reprocessing system (2) described above, it should be understood that detection system (300) may be incorporated into various other kinds of reprocessing systems. By way of example only, reprocessing system (2) may be readily incorporated into any of the various reprocessing systems described in U.S. Patent App. No. 15/157650, entitled "Apparatus and Method for Reprocessing a Medical Device," filed on even date herewith.

As discussed above, flow rates in supply lines (30) can be monitored via channel pumps (32) and pressure sensors (42) as well as level indicating sensors (138) of metering tube (136). As shown in FIG. 4, a flow sensor (308) may also be provided to monitor the flow rates of fluid entering endoscope (302). It should be understood that flow sensor (308) may be provided in lieu of sensor (42) and/or sensor (138). In other words, sensors (42, 138) may be omitted in some versions. In the present example, flow sensor (308) is incorporated with a main fluid line (310) for providing fluid to channels (304, 306) of endoscope (302). As fluid passes through flow sensor (308), information regarding the flow rate is captured by flow sensor (308) and communicated to control system (20) for use in detecting the type of endoscope (302) disposed in contamination basin (14).

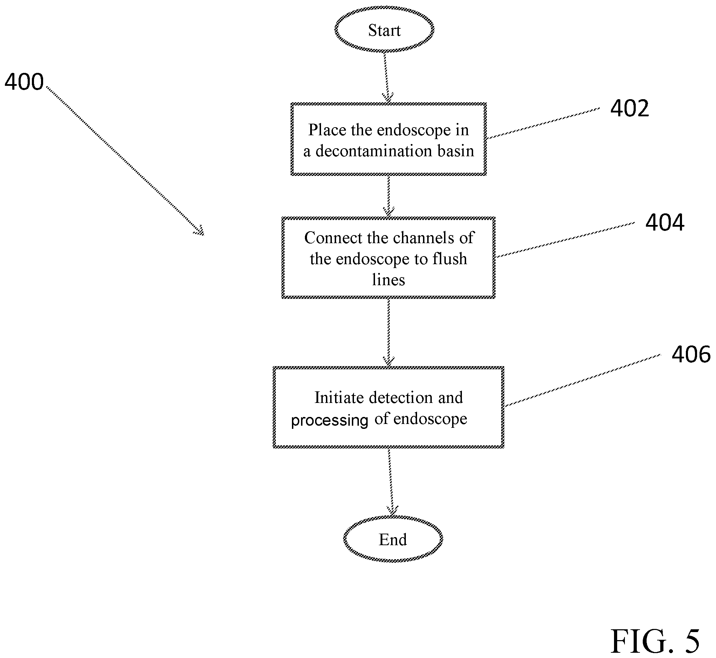

As shown in FIGS. 4 and 5, in some versions of detection system (300) and a method (400) of using detection system (300), a user places endoscope (302) into one of the decontamination basins (14a, 14b). This initial step is shown in a step (402) of FIG. 5. Thereafter, step (402) moves to a step (404), where the user connects endoscope (302) with reprocessing system (2). After endoscope (302) is connected to detection system (300), step (404) moves to a step (406) where control system (20) initiates a detection routine for detecting the type of endoscope disposed in contamination basin (14a, 14b) and determining the preferred processing profile for the particular endoscope (302). The detection routine is configured to iteratively test each channel (304, 306) of the underlying endoscope (302) independently of the other channels (304, 306) and collect information regarding the flow of fluid through the particular channel (304, 306).

In order to test a single channel (304, 306), detection system (300) is configured to close the unselected channels (304, 306) by closing a valve associated with the particular channel. For example, as shown in FIG. 4, a first valve (314) is associated with first channel (304) and a second valve (316) is associated with second channel (306). First valve (314) and second valve (316) may be incorporated into the corresponding connector (31) of the associated flush line (30) and electrically coupled with control unit (20) to allow control unit (20) to selectively open and close the first valve (314) and second valve (316) as needed by detection system (300). While first valve (314) and second valve (316) are illustrated as incorporated into the corresponding connector (31), first valve (314) and second valve (316) may be disposed along any portion of reprocessing system (2) that allows detection system (300) to selectively open and close fluid flow independently for channels (304, 306) using first valve (314) and second valve (316). For example, in other versions of detection system (300), first valve (314) may be disposed downstream of flow sensor (308) and upstream of channel pump (32). When detection system (300) tests the fluid flow of first channel (304), fluid is prevented from entering all other channels (306) by closing the valves (316) associated with the other channels (306). For example, fluid is prevented from entering second channel (306) by closing second valve (316).

As each channel (304, 306) is tested separately, the fluid flow information regarding all channels (304, 306) is collected. Once every channel (304, 306) is tested, the collected fluid flow information is compiled and used to determine the type or style or overall requirements of the underlying endoscope (302). The determination may be made by comparing the collected information to a lookup table or a database or other information stored in a memory accessible by control system (20). After the particular type of endoscope (302) is identified, a processing profile is retrieved for the particular endoscope (302) and reprocessing system (2) proceeds to clean and disinfect endoscope (302) in accordance with the corresponding processing profile. In some versions, the memory that is accessible by control system (20) stores a certain number of processing profiles, such that reprocessing system (2) selects the best fit processing profile from the pre-existing set of processing profiles based on the data obtained using detection system (300). In some other versions, control system (20) is able to generate an ad hoc processing profile based on the data obtained using detection system (300).

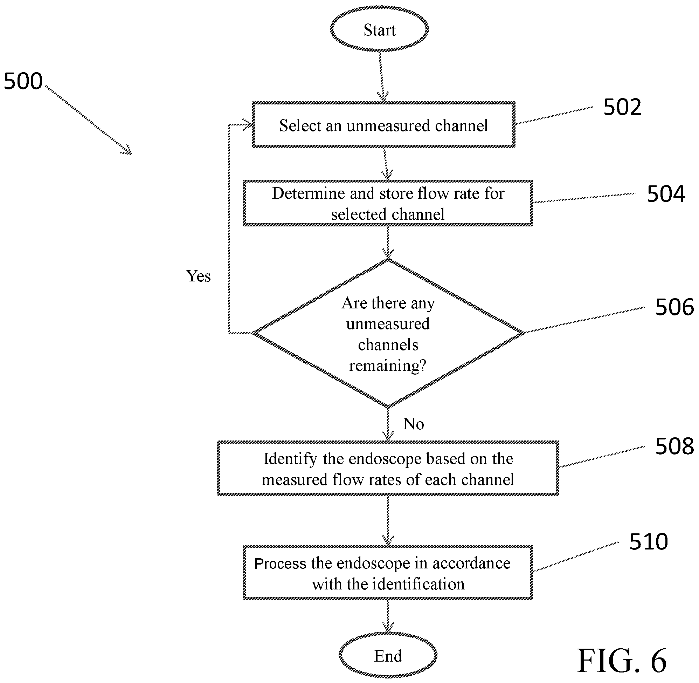

For example, and with reference to FIGS. 4 and 5, in some versions of detection system (300) and a method (500) of utilizing detection system (300), the detection routine first selects an unmeasured channel (304, 306) such as channel (304), and tests channel (304) of endoscope (302) by pumping fluid into first channel (304) at a particular set pressure. This step is illustrated as step (502) of FIG. 6. At this stage, valve (316) is in a closed state while valve (314) is in an open state, such that channel (304) is the only channel (304, 306) through which fluid is flowing. As shown in a step (504), as fluid is pumped into first channel (304), flow sensor (308) monitors the flow of fluid passing therethrough, such that data from flow sensor (308) will be indicative of the fluid flow through first channel (304). At the completion of the test of first channel (304), flow sensor (308) provides the collected information regarding the total fluid flow to control system (20).

Next, method (500) determines whether there are any unmeasured channels (304, 306) remaining, as shown in step (506). If there are unmeasured channels (304, 306) remaining, the detection routine loops back to step (502) and selects another unmeasured channel (304, 306) such as channel (306) and tests second channel (306) by pumping fluid into second channel (306) at a particular set pressure. At this stage, valve (314) is in a closed state while valve (316) is in an open state, such that channel (306) is the only channel (304, 306) through which fluid is flowing. Flow sensor (308) collects information regarding the total fluid flow through second channel (306) during the testing of second channel (306) and provides this information to control system (20). In versions where endoscope (302) includes more than two channels (304, 306), the flow through each channel may be discretely tested by selectively opening and closing per-channel valves in a sequence in accordance with the above teachings.

When there are no remaining unmeasured channels (304, 306), step (506) proceeds to a step (508). In step (508), control system (20) compares the fluid flow for each channel (304, 306) with corresponding endoscope profile information accessible by control system (20). Upon finding an endoscope profile having a matching number of channels with matching fluid flow characteristics, step (508) proceeds to a step (510), whereby control system (20) proceeds to clean and disinfect the underlying endoscope (302) according to a stored processing profile for the matching dataset. Alternatively, as noted above, control system (20) may be configured to generate an ad hoc processing profile based on the fluid flow data from flow sensor (308). In such versions, control system (20) does not necessarily need to have a set of predefined processing profiles stored.

In some versions of detection system (300), fluid flow characteristics are described and utilized as a mechanism for collecting information regarding the various channels (302, 306) of the underlying endoscope (302). However, in other versions of detection system (300), the volume of fluid entering a channel (302, 306) may be constant, and the resulting pressure may be measured (e.g., using pressure sensors (42)) and used as the mechanism for collecting information regarding the channel (302, 306). Furthermore, any other metric such as fluid flow or pressure may be used as a mechanism for collecting information regarding channels (302, 306) of endoscope (302).

With specific reference to duodenoscopes, inasmuch as open elevator channels (306) allow a relatively small flow of fluid to pass therethrough at a given pressure, detection system (300) may be configured to determine whether a particular channel (304, 306) is an open elevator channel (306) based on the flow of fluid passing through flow sensor (308). Accordingly, detection system (300) may include a threshold fluid flow amount for indicating when a particular channel (304, 306) is an open elevator channel (306). Similarly, detection of an open elevator channel (306) indicates the underlying endoscope (302) is a duodenoscope, and therefore control system (20) may take additional processing steps to accommodate cleaning and/or disinfecting of a duodenoscope in general, or an open elevator channel (306) duodenoscope specifically, such as an extended flow and purge time for properly disinfecting the open elevator channel (306).

A dedicated flow sensor (312) with low flow detection capability can be used in combination with flow sensor (308) to confirm the measurements of flow sensor (308). As shown in FIG. 4, dedicated flow sensor (312) may be positioned on a particular flush line (30). In some versions of detection system (300), the particular flush line (30) may be specifically intended for connecting with elevator channels (306) of duodenoscopes. In these versions, detection system (300) thereby provides an enhanced detection and flow monitoring capability to elevator channel (306). In some other versions, each and every flush line has its own dedicated flow sensor (312), such that data from a combination of flow sensors (308, 312) may be used to determine the characteristics of endoscope (302) and thereby select or generate a processing profile that is best suited for the particular endoscope in basin (14a, 14b).

Detection system (300) may be configured to run an error check routine in conjunction with the initiation routine to address the possibility that a valve (314, 316) could become stuck or otherwise remain open during the testing of channels (304, 306) and therefore give a false determination of the type of the underlying endoscope (302). For example, if first valve (314) associated with first channel (304) is stuck in an open position while detection system (300) is testing second channel (306), an increased fluid flow rate will be sensed by flow sensor (308) due to the extra fluid traveling into first channel (304).

Detection system (300) begins the error check routine by closing all valves (314, 316) disposed between flow sensor (308) and the corresponding channels (304, 306) of the underlying endoscope (302). In the example shown in FIG. 4, the error check routine closes first valve (314) and second valve (316). Thereafter, the error check routine allows fluid to pass from main line (310) through flow sensor (308) and to first valve (314) and second valve (316). If both valves are legitimately closed, no fluid can pass therethrough and flow sensor (308) does not detect any flow of fluid through flow sensor (308). If a particular valve (314, 316) is stuck in an open position, fluid will pass through the malfunctioning valve (314, 316) and flow sensor (308) will detect a flow of fluid. Upon detection of a flow of fluid, control unit (20) is alerted and corrective steps are taken to address the malfunction. For instance, an alarm may be sounded to alert the user that a problem has occurred and a valve (314, 316) is malfunctioning or an alternative processing profile may be used to account for a malfunctioning valve (314, 316). If the error check routine indicates that the underlying valves (314, 316) are working properly, control unit (20) may proceed in cleaning and disinfecting the underlying endoscope (302) in accordance with the selected or generated processing profile with the assurance that the endoscope (302) type was accurately determined by the detection routine.

### V. Miscellaneous

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus (2) for processing a medical instrument by passing one or both of a detergent and a disinfectant through a plurality of channels (304, 306) defined by the medical instrument (302), wherein the apparatus comprises:
(a) a detection system (300) configured to automatically detect a type of instrument disposed in the apparatus, the detection system (300) configured to collect information regarding the plurality of channels (304, 306) of the medical instrument (302) and determine a fluid parameter for each channel of the plurality of channels (304, 306);
(b) a set of instrument profiles stored in a memory; and
(c) a control system (20) configured to identify, based at least in part on the fluid parameter for each channel in the plurality of channels (304, 306), an instrument profile associated with the medical instrument (302), and pass one or both of a detergent and a disinfectant through the channels (304, 306) of the medical instrument (302) based at least in part on the identified instrument profile selected from the set of instrument profiles.

2. The apparatus of claim 1, wherein the detection system (300) further comprises a sensor (308) configured to collect information regarding each channel in the plurality of channels (304, 306).

3. The apparatus of claim 2, wherein the sensor (308) is configured to determine the flow rate of fluid passing through each channel in the plurality of channels (304, 306) at a set pressure.

4. The apparatus of claim 3, wherein the detection system (300) further comprises a low flow sensor (312), wherein the low flow sensor (312) is dedicated to a selected channel (306) in the plurality of channels.

5. The apparatus of any one or more of claims 2 to 4, wherein the sensor (308) is configured to determine the flow rate of fluid through each channel in the plurality of channels (304, 306) when a set volume of fluid is passed therethrough.

6. The apparatus of any one or more of claims 1 to 5, further comprising a set of processing profiles, wherein each instrument profile in the set of instrument profiles is associated with a corresponding processing profile in the set of processing profiles.

7. The apparatus of claim 6, wherein the control system (20) is configured to process the medical instrument (302) based at least in part on the processing profile associated with the selected instrument profile.

8. The apparatus of any one or more of claims 1 to 7, wherein the detection system (300) further comprises a plurality of valves (314, 316) in communication with the control system (20), wherein each valve in the plurality of valves (314, 316) is associated with a corresponding channel in the plurality of channels (304, 306), wherein each valve in the plurality of valves (314, 316) is operable to be open and closed by the control system (20).

9. The apparatus of claim 8, wherein one of the control system (20) or the detection system (300) is configured to actuate at least one valve in the plurality of valves (314, 316) in preparation of collecting information regarding a particular channel in the plurality of channels (304, 306).

10. The apparatus of any one or more of claims 8 or 9, wherein the detection system (300) is configured to determine whether any valve in the plurality of valves (314, 316) are malfunctioning.

11. The apparatus of claim 10, wherein to determine whether any valve in the plurality of valves (314, 316) are malfunctioning, the detection system (300) is configured to:
(i) initiate a closure command to each valve in the plurality of valves (314, 316), and
(ii) determine whether each valve in the plurality of valves (314, 316) is closed.

12. The apparatus of any one or more of claims 10 to 11, wherein each valve in the plurality of valves (314, 316) is disposed in a corresponding connector (31) in a plurality of connectors, wherein each connector (31) in the plurality of connectors is configured to be connected to at least one of the channels in the plurality of channels (304, 306).

13. The apparatus of any one or more of claims 1 to 12, wherein the control system (20) is configured to identify an open elevator channel in the plurality of channels (304, 306).

14. The apparatus of any one or more of claims 1 to 13, further comprising a pump (32) configured to supply a fluid to each channel in the plurality of channels (304, 306).

15. A method for automatically detecting a type of instrument (302) disposed in a medical instrument processing apparatus (2), the method comprising:
(a) determining a fluid parameter for each channel in a plurality of channels (304, 306) defined by an instrument (302);
(b) identifying, based at least in part on the fluid parameter for each channel in the plurality of channels (304, 306), an instrument profile associated with the medical instrument (302) and stored in a memory; and
(c) performing one or both of cleaning or disinfecting the channels (304, 306) of the medical instrument (302) based at least in part on the identified instrument profile.

16. The method of claim 15, further comprising:
(a) selecting a channel in the plurality of channels (304, 306);
(b) preventing fluid from entering the unselected channels;
(c) allowing fluid to travel through the selected channel; and
(d) collecting information regarding the flow rate of fluid traveling through the selected channel.

17. The method of claim 16, further comprising:
(a) identifying the selected channel as an open elevator channel based at least in part on the collected information regarding the fluid traveling into the selected channel; and
(b) processing the selected channel as an open elevator channel.

18. The method of any one or more of claims 15 to 17, further comprising:
(a) selecting a processing profile in a plurality of processing profiles based at least in part on the identified instrument profile; and
(b) processing the medical instrument (302) based at least in part on the selected processing profile.

## Patentansprüche

1. Vorrichtung (2) zum Verarbeiten eines medizinischen Instruments durch Leiten von einem oder beidem eines Waschmittels und eines Desinfektionsmittels durch eine Vielzahl von Kanälen (304, 306), die durch das medizinische Instrument (302) definiert sind, wobei die Vorrichtung Folgendes umfasst:
(a) ein Detektionssystem (300), das dazu ausgelegt ist, einen Typ von Instrument, das in der Vorrichtung angeordnet ist, automatisch zu detektieren, wobei das Detektionssystem (300) dazu ausgelegt ist, Informationen zu der Vielzahl von Kanälen (304, 306) des medizinischen Instruments (302) zu sammeln und für jeden Kanal der Vielzahl von Kanälen (304, 306) einen Fluidparameter zu bestimmen;
(b) einen Satz von Instrumentenprofilen, der in einem Speicher gespeichert ist; und
(c) ein Steuersystem (20), das dazu ausgelegt ist, mindestens teilweise auf Basis des Fluidparameters für jeden Kanal in der Vielzahl von Kanälen (304, 306) ein Instrumentenprofil zu identifizieren, das mit dem medizinischen Instrument (302) verknüpft ist, und mindestens teilweise auf Basis des identifizierten Instrumentenprofils, das aus dem Satz von Instrumentenprofilen ausgewählt wurde, eines oder beides eines Waschmittels und eines Desinfektionsmittels durch die Kanäle (304, 306) des medizinischen Instruments (302) zu leiten.

2. Vorrichtung nach Anspruch 1, wobei das Detektionssystem (300) ferner einen Sensor (308) umfasst, der dazu ausgelegt ist, Informationen zu jedem Kanal in der Vielzahl von Kanälen (304, 306) zu sammeln.

3. Vorrichtung nach Anspruch 2, wobei der Sensor (308) dazu ausgelegt ist, die Durchflussrate von Fluid, das mit einem eingestellten Druck durch jeden Kanal in der Vielzahl von Kanälen (304, 306) geleitet wird, zu bestimmen.

4. Vorrichtung nach Anspruch 3, wobei das Detektionssystem (300) ferner einen Sensor (312) für geringen Durchfluss umfasst, wobei der Sensor (312) für geringen Durchfluss für einen ausgewählten Kanal (306) in der Vielzahl von Kanälen dediziert ist.

5. Vorrichtung nach einem oder mehreren der Ansprüche 2 bis 4, wobei der Sensor (308) dazu ausgelegt ist, die Durchflussrate von Fluid durch jeden Kanal in der Vielzahl von Kanälen (304, 306) zu bestimmen, wenn ein eingestelltes Fluidvolumen dadurch geleitet wird.

6. Vorrichtung nach einem oder mehreren der Ansprüche 1 bis 5, die ferner einen Satz von Verarbeitungsprofilen umfasst, wobei jedes Instrumentenprofil im Satz von Instrumentenprofilen mit einem entsprechenden Verarbeitungsprofil im Satz von Verarbeitungsprofilen verknüpft ist.

7. Vorrichtung nach Anspruch 6, wobei das Steuersystem (20) dazu ausgelegt ist, das medizinische Instrument (302) mindestens teilweise auf Basis des Verarbeitungsprofils, das mit dem ausgewählten Instrumentenprofil verknüpft ist, zu verarbeiten.

8. Vorrichtung nach einem oder mehreren der Ansprüche 1 bis 7, wobei das Detektionssystem (300) ferner eine Vielzahl von Ventilen (314, 316) in Kommunikation mit dem Steuersystem (20) umfasst, wobei jedes Ventil in der Vielzahl von Ventilen (314, 316) mit einem entsprechenden Kanal in der Vielzahl von Kanälen (304, 306) verknüpft ist, wobei jedes Ventil in der Vielzahl von Ventilen (314, 316) betreibbar ist, vom Steuersystem (20) geöffnet und geschlossen zu werden.

9. Vorrichtung nach Anspruch 8, wobei eines vom Steuersystem (20) oder vom Detektionssystem (300) dazu ausgelegt ist, zur Vorbereitung des Sammelns von Informationen zu einem bestimmten Kanal in der Vielzahl von Kanälen (304, 306) mindestens ein Ventil in der Vielzahl von Ventilen (314, 316) zu betätigen.

10. Vorrichtung nach einem oder mehreren der Ansprüche 8 oder 9, wobei das Detektionssystem (300) dazu ausgelegt ist zu bestimmen, ob eines der Ventile in der Vielzahl von Ventilen (314, 316) nicht korrekt funktioniert.

11. Vorrichtung nach Anspruch 10, wobei das Detektionssystem (300) zum Bestimmen, ob ein Ventil in der Vielzahl von Ventilen (314, 316) nicht korrekt funktioniert, zu Folgendem ausgelegt ist:
(i) Initiieren eines Schließbefehls an jedes Ventil in der Vielzahl von Ventilen (314, 316) und
(ii) Bestimmen, ob jedes Ventil in der Vielzahl von Ventilen (314, 316) geschlossen ist.

12. Vorrichtung nach einem oder mehreren der Ansprüche 10 bis 11, wobei jedes Ventil in der Vielzahl von Ventilen (314, 316) in einem entsprechenden Verbinder (31) in einer Vielzahl von Verbindern angeordnet ist, wobei jeder Verbinder (31) in der Vielzahl von Verbindern dazu ausgelegt ist, mit mindestens einem der Kanäle in der Vielzahl von Kanälen (304, 306) verbunden zu werden.

13. Vorrichtung nach einem oder mehreren der Ansprüche 1 bis 12, wobei das Steuersystem (20) dazu ausgelegt ist, einen offenen Aufzugkanal in der Vielzahl von Kanälen (304, 306) zu identifizieren.

14. Vorrichtung nach einem oder mehreren der Ansprüche 1 bis 13, die ferner eine Pumpe (32) umfasst, die dazu ausgelegt ist, jedem Kanal in der Vielzahl von Kanälen (304, 306) ein Fluid zuzuführen.

15. Verfahren zum automatischen Detektieren eines Typs von Instrument (302), das in einer Verarbeitungsvorrichtung (2) für medizinische Instrumente angeordnet ist, wobei das Verfahren Folgendes umfasst:
(a) Bestimmen eines Fluidparameters für jeden Kanal in einer Vielzahl von Kanälen (304, 306), die durch ein Instrument (302) definiert sind;
(b) Identifizieren eines Instrumentenprofils, das mit dem medizinischen Instrument (302) verknüpft und in einem Speicher gespeichert ist, mindestens teilweise auf Basis des Fluidparameters für jeden Kanal in der Vielzahl von Kanälen (304, 306); und
(c) Durchführen von einem oder beidem eines Reinigens oder eines Desinfizierens der Kanäle (304, 306) des medizinischen Instruments (302) mindestens teilweise auf Basis des identifizierten Instrumentenprofils.

16. Verfahren nach Anspruch 15, das ferner Folgendes umfasst:
(a) Auswählen eines Kanals in der Vielzahl von Kanälen (304, 306);
(b) Verhindern, dass Fluid in die nicht ausgewählten Kanäle eintritt;
(c) Zulassen, dass Fluid durch den ausgewählten Kanal strömt; und
(d) Sammeln von Informationen zur Durchflussrate von Fluid, das durch den ausgewählten Kanal strömt.

17. Verfahren nach Anspruch 16, das ferner Folgendes umfasst:
(a) Identifizieren des ausgewählten Kanals mindestens teilweise auf Basis der gesammelten Informationen zum Fluid, das in den ausgewählten Kanal strömt, als einen offenen Aufzugkanal und
(b) Verarbeiten des ausgewählten Kanals als einen offenen Aufzugkanal.

18. Verfahren nach einem oder mehreren der Ansprüche 15 bis 17, das ferner Folgendes umfasst:
(a) Auswählen eines Verarbeitungsprofils in einer Vielzahl von Verarbeitungsprofilen mindestens teilweise auf Basis des identifizierten Instrumentenprofils und
(b) Verarbeiten des medizinischen Instruments (302) mindestens teilweise auf Basis des ausgewählten Verarbeitungsprofils.

## Revendications

1. Appareil (2) pour traiter un instrument médical en faisant passer l'un d'un détergent et d'un désinfectant ou les deux à travers une pluralité de canaux (304, 306) définis par l'instrument médical (302), l'appareil comprenant :
(a) un système de détection (300) configuré pour détecter automatiquement un type d'instrument disposé dans l'appareil, le système de détection (300) étant configuré pour recueillir des informations concernant la pluralité de canaux (304, 306) de l'instrument médical (302) et déterminer un paramètre de fluide pour chaque canal de la pluralité de canaux (304, 306) ;
(b) un ensemble de profils d'instrument stockés dans une mémoire ; et
(c) un système de commande (20) configuré pour identifier, d'après au moins en partie le paramètre de fluide pour chaque canal dans la pluralité de canaux (304, 306), un profil d'instrument associé à l'instrument médical (302), et faire passer l'un d'un détergent et d'un désinfectant ou les deux à travers les canaux (304, 306) de l'instrument médical (302) d'après au moins en partie le profil d'instrument identifié sélectionné parmi l'ensemble de profils d'instrument.

2. Appareil selon la revendication 1, dans lequel le système de détection (300) comprend en outre un capteur (308) configuré pour recueillir des informations concernant chaque canal dans la pluralité de canaux (304, 306) .

3. Appareil selon la revendication 2, dans lequel le capteur (308) est configuré pour déterminer le débit de fluide passant à travers chaque canal dans la pluralité de canaux (304, 306) à une pression établie.

4. Appareil selon la revendication 3, dans lequel le système de détection (300) comprend en outre un capteur de faible débit (312), dans lequel le capteur de faible débit (312) est dédié à un canal sélectionné (306) dans la pluralité de canaux.

5. Appareil selon l'une quelconque ou plusieurs des revendications 2 à 4, dans lequel le capteur (308) est configuré pour déterminer le débit de fluide à travers chaque canal dans la pluralité de canaux (304, 306) lorsqu'un volume établi de fluide passe au travers.

6. Appareil selon l'une quelconque ou plusieurs des revendications 1 à 5, comprenant en outre un ensemble de profils de traitement, dans lequel chaque profil d'instrument dans l'ensemble de profils d'instrument est associé à un profil de traitement correspondant dans l'ensemble de profils de traitement.

7. Appareil selon la revendication 6, dans lequel le système de commande (20) est configuré pour traiter l'instrument médical (302) d'après au moins en partie le profil de traitement associé au profil d'instrument sélectionné.

8. Appareil selon l'une quelconque ou plusieurs des revendications 1 à 7, dans lequel le système de détection (300) comprend en outre une pluralité de vannes (314, 316) en communication avec le système de commande (20), dans lequel chaque vanne dans la pluralité de vannes (314, 316) est associée à un canal correspondant dans la pluralité de canaux (304, 306), dans lequel chaque vanne dans la pluralité de vannes (314, 316) est opérationnelle pour être ouverte et fermée par le système de commande (20).

9. Appareil selon la revendication 8, dans lequel l'un du système de commande (20) ou du système de détection (300) est configuré pour actionner au moins une vanne dans la pluralité de vannes (314, 316) en préparation à un recueil d'informations concernant un canal particulier dans la pluralité de canaux (304, 306).

10. Appareil selon l'une quelconque ou plusieurs des revendications 8 et 9, dans lequel le système de détection (300) est configuré pour déterminer si une vanne quelconque dans la pluralité de vannes (314, 316) fonctionne mal.

11. Appareil selon la revendication 10, dans lequel pour déterminer si une vanne quelconque dans la pluralité de vannes (314, 316) fonctionne mal, le système de détection (300) est configuré pour :
(i) lancer un ordre de fermeture à chaque vanne dans la pluralité de vannes (314, 316), et
(ii) déterminer si chaque vanne dans la pluralité de vannes (314, 316) est fermée.

12. Appareil selon l'une quelconque ou plusieurs des revendications 10 et 11, dans lequel chaque vanne dans la pluralité de vannes (314, 316) est disposée dans un raccord (31) correspondant dans une pluralité de raccords, dans lequel chaque raccord (31) dans la pluralité de raccords est configuré pour être raccordé à au moins l'un des canaux dans la pluralité de canaux (304, 306).

13. Appareil selon l'une quelconque ou plusieurs des revendications 1 à 12, dans lequel le système de commande (20) est configuré pour identifier un canal élévateur ouvert dans la pluralité de canaux (304, 306).

14. Appareil selon l'une quelconque ou plusieurs des revendications 1 à 13, comprenant en outre une pompe (32) configurée pour fournir un fluide à chaque canal dans la pluralité de canaux (304, 306).

15. Procédé de détection automatique d'un type d'instrument (302) disposé dans un appareil de traitement d'instrument médical (2), le procédé comprenant :
(a) la détermination d'un paramètre de fluide pour chaque canal dans la pluralité de canaux (304, 306) définis par un instrument (302) ;
(b) l'identification, d'après au moins en partie le paramètre de fluide pour chaque canal dans la pluralité de canaux (304, 306), d'un profil d'instrument associé à l'instrument médical (302) et stocké dans une mémoire ; et
(c) la réalisation de l'un du nettoyage ou de la désinfection ou des deux des canaux (304, 306) de l'instrument médical (302) d'après au moins en partie le profil d'instrument identifié.

16. Procédé selon la revendication 15, comprenant en outre :
(a) la sélection d'un canal dans la pluralité de canaux (304, 306) ;
(b) le fait d'empêcher un fluide d'entrer dans les canaux non sélectionnés ;
(c) le fait d'autoriser le fluide à circuler à travers le canal sélectionné ; et
(d) le recueil d'informations concernant le débit de fluide circulant à travers le canal sélectionné.

17. Procédé selon la revendication 16, comprenant en outre :
(a) l'identification du canal sélectionné en tant que canal élévateur ouvert d'après au moins en partie les informations recueillies concernant le fluide circulant dans le canal sélectionné ; et
(b) le traitement du canal sélectionné en tant que canal élévateur ouvert.

18. Procédé selon l'une quelconque ou plusieurs des revendications 15 à 17, comprenant en outre :
(a) la sélection d'un profil de traitement dans une pluralité de profils de traitement d'après au moins en partie le profil d'instrument identifié ; et
(b) le traitement de l'instrument médical (302) d'après au moins en partie le profil de traitement sélectionné.
